(19) Europäisches Patentamt
European Patent Office
Office européen des brevets

(11) Numéro de publication: **0 305 270**
**A1**

(12) # DEMANDE DE BREVET EUROPEEN

(21) Numéro de dépôt: **88402102.3**

(22) Date de dépôt: **12.08.88**

(51) Int. Cl.[4]: **C 07 D 233/78**
C 07 D 233/88, A 61 K 31/415

(30) Priorité: **13.08.87 FR 8711544**

(43) Date de publication de la demande:
**01.03.89 Bulletin 89/09**

(84) Etats contractants désignés:
**AT BE CH DE FR GB IT LI LU NL SE**

(71) Demandeur: **ROUSSEL-UCLAF**
**35, boulevard des Invalides**
**F-75007 Paris (FR)**

(72) Inventeur: **Coussediere, Daniel**
**186, Boulevard de l'Europe**
**F-93370 Montfermeil (FR)**

**Gigliotti, Giuseppe**
**5, Rue Alphonse Baudin Appt. 464**
**F-75011 Paris (FR)**

**Moguilewski, Martine**
**37, Rue Lamartine**
**F-75009 Paris (FR)**

(74) Mandataire: **Bourgouin, André et al**
**Département des Brevets ROUSSEL UCLAF 111, route de Noisy B.P. no 9**
**F-93230 Romainville (FR)**

(54) **Nouvelles imidazolidines substitués par un radical hydroxyméthyle et un radical phényl substitue,leur procédé de préparation, leur application comme médicaments, les compositions pharmaceutiques les renfermant et un intermédiaire pour leur préparation.**

(57) L'invention a pour objet les produits de formule générale I :

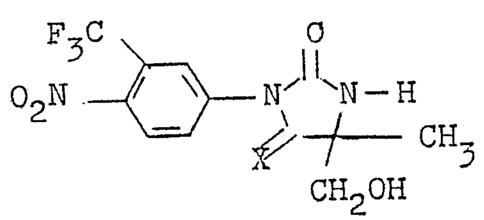

dans laquelle X représente un atome d'oxygène ou un groupement imino sous toutes leurs formes racémique et optiquement actives possibles, leur procédé de préparation, leur application comme médicaments, les compositions pharmaceutiques les renfermant et un intermédiaire pour leur préparation.

EP 0 305 270 A1

## Description

**Nouvelles imidazolidines substitués par un radical hydroxyméthyle et un radical phényl substitué, leur procédé de préparation, leur application comme médicaments, les compositions pharmaceutiques les renfermant et un intermédiaire pour leur préparation.**

La présente invention a pour objet de nouvelles imidazolidines substituées par un radical hydroxyméthyle et un radical phényle substitué, leur procédé de préparation, leur application comme médicamments, les compositions pharmaceutiques les renfermant et un intermédiaire pour leur préparation.

La 1-(3′-trifluorométhyl 4′-nitro phényl) 4,4-diméthyl imidazolidine 2,5-dione de formule :

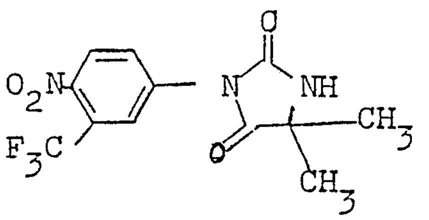

a été décrite dans le brevet francais B.F 2.329.276 publié le 27 mai 1977.

Le produit dont le nom de code est RU 23908, la dénomination commune internationale NILUTAMIDE et le nom de marque ANANDRON[R] à fait l'objet de nombreuses publications.

Dans un article intitulé "Pharmacology and Clinical Studies with 23908" (ANANDRON[R]) paru dans l'ouvrage EORTC Genitourinary Group Monograph 2, part A : Therapeutic Principles in Metastatic Prostatic Cancer pages 99 - 120, 1985, 1es auteurs J.P. RAYNAUD et al ont notamment décrit les métabolites supposés du 23908 chez le rat. Ces métabolites dont le groupement $NO_2$ porté sur le noyau phényle a été réduit en NHOH ou en $NH_2$ se sont tous révélés dénués d'activité anti-androgène.

Or la demanderesse vient de découvrir de facon surprenante que chez l'homme un métabolite jusqu'ici inconnu du 23908 présente une très intéressante activité anti-androgène. Il s'agit du produit de formule A

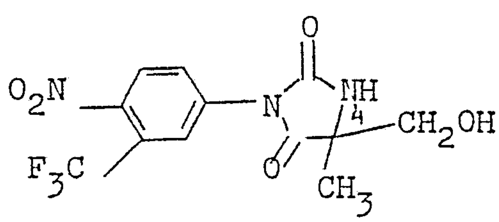

A

La société demanderesse a préparé ce produit par voie chimique en utilisant comme intermédiaire le produit de formule B

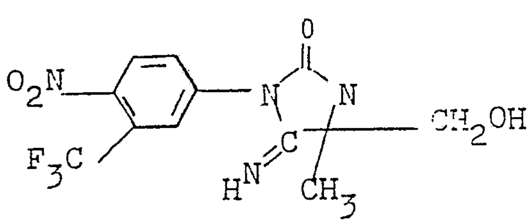

B

La présente demande a donc pour objet les produits de formule générale I

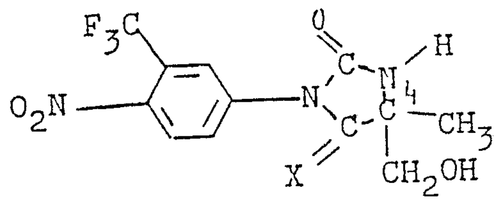

I

dans laquelle X représente un atome d'oxygène ou un groupement imine sous toutes leurs formes racémique et optiquement actives possibles.

Cette formule regroupe les produits A et B indiqués ci-dessus. Ces produits qui possèdent en position 4 un carbone asymétrique peuvent se présenter sous forme de racémique c'est à dire de mélange équimoléculaire des deux isomères, sous forme de l'un des isomères 4R ou 4S ou d'un mélange non équimoléculaire de ces deux isomères.

La présente demande a donc pour objet le produit B c'est à dire la 4-(hydroxyméthyl) 5-imino 4-méthyl-1-[4-nitro 3-(trifluorométhyl)phényl] 2-imidazolidinone sous forme racémique ou optiquement active, le produit A c'est à dire la 4-(hydroxyméthyl) 4-méthyl 1-[4-nitro 3-(trifluorométhyl) phényl] 2,5-imidazolidine dione sous forme racémique ou optiquement active et notamment la forme racémique.

La présente demande a également pour objet les produits A sous forme de ses isomères optiquement actifs c'est à dire la (4R) 4-(hydroxyméthyl) 4-méthyl 1-[4-nitro 3-(trifluorométhyl)phényl] 2,5-imidazolidine dione et la (4S) 4-(hydroxyméthyl) 4-méthyl 1-[4-nitro 3-(trifluorométhyl)phényl] 2,5-imidazolidine dione.

Enfin, à titre de produit, la présente demande a pour objet la 4-(hydroxyméthyl) 4-méthyl 1-[4-nitro 3-(trifluorométhyl)phényl] 2,5-imidazolidine dione sous forme d'un mélange des isomères 4R et 4S c'est à dire un mélange différent du mélange 50-50 du racémique.

La présente invention a également pour objet un procédé de préparation des produits de formule I telle que définie ci-dessus caractérisé en ce que l'on fait réagir le 2-amino 2-(hydroxyméthyl) propanenitrile sous forme racémique ou optiquement active avec l'isocyanate de 3-trifluorométhyl-4-nitrophényle pour obtenir un produit de formule I dans laquelle X représente un groupement imino c'est à dire la 4-(hydroxyméthyl) 5-imino 4-méthyl 1-[4-nitro 3-(trifluorométhyl) phényl] 2-imidazolidinone sous forme racémique ou optiquement active, produit de formule I dans laquelle X représente un groupement imino sous forme racémique que, si désiré l'on sépare en ses deux isomères optiquement actifs, et produit de formule I dans laquelle X représente un groupement imino sous forme racémique ou optiquement active que si désiré l'on hydrolyse pour obtenir le produit de formule générale I dans laquelle X représente un atome d'oxygène, c'est à dire la 4-(hydroxyméthyl) 4-méthyl 1-[4-nitro 3-(trifluorométhyl) phényl] 2,5-imidazolidine-dione sous forme racémique ou optiquement active et produit de formule I dans laquelle X représente un atome d'oxygène sous forme racémique que si désiré l'on sépare en ses isomères optiquement actifs.

Dans un mode préféré d'exécution du procédé ci-dessus, la réaction entre le 2-amino 2(hydroxyméthyl) propanenitrile et l'isocyanate de 3-trifluorométhyl 4-nitrophényle est effectuée dans un solvant tel que, de préférence, le dichloréthane, mais on peut également utiliser le tétrahydrofuranne, l'éther éthylique ou isopropylique.

On opère de préférence à température ambiante sans addition d'autre réactif.

Dans une variante du procédé ci-dessus, on peut également utiliser le 2-amino 2-(hydroxyméthyl) propane nitrile dont le radical hydroxy est protégé par un groupement R facilement éliminable, notamment par hydrolyse acide.

On obtient après action avec l'isocyanate de 3-trifluorométhyl 4-nitrophényle un composé intermédiaire de formule

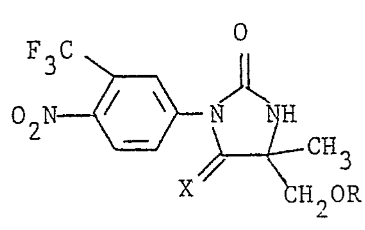

dans laquelle X représente un radical imino et R représente un groupement protecteur du radical hydroxy, composé que l'on soumet à une hydrolyse acide pour obtenir un composé de formule I dans laquelle X représente un atome d'oxygène.

Parmi les valeurs de R, on peut citer par exemple les groupements trityle, silyle, terbutyle, ainsi que les groupements de formule

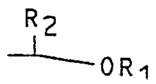

dans lesquels $R_1$ et $R_2$ représentent un radical alkyle linéaire ou cyclique éventuellement substitué et plus particulièrement le radical méthoxyméthyle, tétrahydrofuryle ou tétrahydropyrannyle.

Dans des conditions préférentielles de mise en oeuvre du procédé de la variante :
- le groupement protecteur du radical hydroxy est le groupement tétrahydropyrannyle,
- l'hydrolyse acide est effectuée au moyen d'acide chlorhydrique en opérant à température du reflux.

Le dédoublement éventuel du produit racémique de formule I dans laquelle X représente un groupement imino, produit racémique qui est obtenu lorsque l'on part d'un produit 2-amino 2(hydroxyméthyl) propanenitrile lui-même racémique, est effectué dans les conditions usuelles . On peut par exemple préparer un hémisuccinate du produit I et dédoubler l'hémisuccinate ainsi obtenu à l'aide d'une base optiquement active telle que l'éphédrine.

On peut également condenser le produit de formule I racémique avec un lactol optiquement actif au sein d'un solvant organique, puis séparer les deux éthers diastéréoisomères selon les méthodes usuelles, par exemple par chromatographie ou par cristallisation et enfin cliver chacun de ces diastéréoisomères en milieu acide pour obtenir les isomères optiquement actifs du produit de formule I.

On peut également, selon la variante du procédé de l'invention, utiliser le 2-amino 2-(hydroxyméthyl) propane nitrile dont le radical hydroxy est protégé par un groupement R de formule

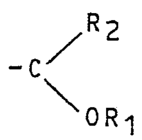

dans laquelle $R_1$ et $R_2$ ont les valeurs indiquées précédemment et possèdant un carbone asymétrique, et ou bien l'on sépare le cas échéant les isomères optiquement actifs et soumet chaque isomère aux réactions indiquées ci-dessus pour obtenir les isomères optiquement actifs du produit de formule I dans lequel X représente un atome d'oxygène ou bien l'on fait réagir avec l'isocyanate de 3-trifluorométhyl 4-nitrophényle pour obtenir le composé intermédiaire dont on sépare le cas échéant, les isomères optiquement actifs puis soumet chaque isomère à une hydrolyse acide pour obtenir les isomères optiquement actifs du produit de formule I dans lequel X représente un atome d'oxygène.

L'hydrolyse du produit de formule I dans laquelle X représente un groupement imino en produit de formule I dans laquelle X représente un atome d'oxygène est effectuée de préference en présence d'un acide tel que l'acide chlorhydrique ou l'acide sulfurique. Le dédoublement éventuel du produit de formule I dans laquelle X représente un atome d'oxygène lorsque celui-ci est sous forme racémique, car il résulte de l'hydrolyse d'un produit racémique de formule I dans laquelle X représente un groupement imino, est effectuée dans les mêmes conditions que le dédoublement de ce même produit. Si l'on part du 2-amino 2-(hydroxyméthyl) propanenitrile sous forme optiquement active on conserve normalement au cours de la synthèse l'isomère optique du produit de départ.

Le 2-amino 2-(hydroxyméthyl) propanenitrile est préparé à partir de l'hydroxyacétone par action du cyanure de sodium, en présence, de préférence de sulfate d'ammonium. Le dédoublement éventuel du produit racémique obtenu est effectué à l'aide d'un acide asymétrique tel que l'acide tartrique ou l'un de ses dérivés tels que les acides dibenzoyltartrique ou paratolyltartrique.

A la connaissance de la demanderesse le 2-amino 2-(hydroxyméthyl) propane nitrile sous forme racémique ou optiquement active est un produit nouveau.

La présente invention a donc également pour objet ce produit à titre de produit industriel nouveau.

Comme le produit RU 23908, les produits de formule I, et en particulier le produit A sous formes racémique ou optiquement actives, sont doués de propriétés pharmacologiques intéressantes ; on a constaté notamment qu'ils inhibaient les effets des androgènes sur les récepteurs périphériques sans être un inhibiteur hypophysaire. Des tests dans la partie expérimentale illustrent chez le rat cette activité anti-androgène.

Du fait de leur activité anti-androgène et de leur innocuité sur le fonctionnement hypophysaire, les composés de formule I peuvent être utilisés en thérapeutique, d'une part chez les adolescents sans craindre un arrêt de croissance, d'autre part chez les adultes sans avoir à redouter certains effets d'une castration chimique.

Les produits de formule I et notamment le produit A sous forme racémique ou optiquement active trouvent donc leur emploi comme médicaments pour le traitement des adénomes et des néoplasies de la prostate, de l'hirsutisme, de l'acné, de la séborrhée et de l'hyperpilosité.

Comme le RU 23908 les produits de formule I et notamment le produit A trouvent leur emploi préférentiel dans le traitement des adénomes et des néoplasies de la prostate.

Comme le RU 23908 les produits de formule I et notamment le produit A peuvent être associés à des peptides du type LH-RH à activité agoniste ou antagoniste dans le traitement de l'adénocarcinome de la prostate et de l'hypertrophie bénigne de la prostate, de l'endométriose, de la dysménorrhée, de l'hirsutisme et des tumeurs mammaires hormonodépendantes. On trouve par exemple une description de ces peptides du type LH RH dans le brevet francais BF 2.465.486.

La présente invention a donc également pour objet un produit comprenant au moins une substance de type LH-RH à activité agoniste ou antagoniste, préférentiellement agoniste, et au moins un produit de formule I, préférentiellement le produit A, en tant que produit de combinaison, pour une utilisation simultanée, séparée ou étalée dans le temps dans le traitement l'adénocarcinome de la prostate, de l'hypertrophie bénigne de la prostate, de l'endométriose, de la dysménorrhée, de l'hirsutisme et des tumeurs mammaires hormonodépendantes préférentiellement de l'adénocarcinome de la prostate.

Par ailleurs, la présence de récepteurs androgènes a été décrite dans les cellules cancéreuses d'un certain nombre d'organes.

On peut citer par exemple les publications suivantes :

- tumeur de la vessie :
Urology 1985, Fév. Vol. 25(2) pp.161-3
- tumeur du cerveau :
J.Neurooncology 1983, Vol. 1(3) pp.179-89
- tumeur du sein :
Cancer 1984, Déc. 1, Vol.54(11) pp.2436-2440
- lymphome :
J.Steroid Biochemistry, 1984, Oct., Vol. 21(4)pp.421-6
- tumeur du rein :
Cancer 1983, August 1, Vol.54(3) pp.477-81
- tumeur du foie :
Br J.Cancer 1983, Déc., Vol 48(6) pp.791-6
- mélanome :
Br J.Dermatol.1982 Nov., Vol.107 suppl.23 pp.54-9
- tumeur des ovaires :
J.Endocrinol.1981 Sept. Vol. 90(3) pp.421-31
- tumeur des testicules :
J. Stéroid Biochem 11, 261-265, 1979
- Cancer du poumon :
Cancer Res. 45, 4206 - 4214, 1985
- tumeur de la tyroïde :
Surgery 96, 996-1000 1984
- carcinome du larynx :
Arch. Oto laryngol 110, 721 - 724, 1984.
- sarcome des tissus mous :
Cancer Res. 40 861-865 1980.

La publication précitée : Cancer 1984 August 1, Vol.54(3) pp. 477-81 décrit l'absence d'activité du Flutamide (2-méthyl N/4-nitro 3-(trifluorométhyl) phényl/propanamide) dans le carcinome rénal.

Les produits de formule I et notamment le produit A sous forme racémique ou optiquement active peuvent donc être employés seuls ou en association pour le traitement de cancers hormono-dépendants et notamment androgéno-dépendants autres que celui de la prostate. On peut aussi citer les cancers atteignant les organes suivants : vessie, cerveau, sein, système lymphatique, rein, foie, peau et ovaires.

Dans les applications indiquées précédemment, les produits de formule I peuvent être administrés par voie parentérale, buccale, perlinguale, percutanée ou rectale. La voie préférée est la voie buccale ou percutanée.

Les compositions pharmaceutiques utilisées peuvent se présenter sous forme de suspensions injectables, de comprimés enrobés, de capsules, de suppositoires, de préparations dermiques ou de formes à libération contrôlée. Ces compositions sont préparées de facon classique. On peut se reporter, par exemple, au brevet francais BF. 2.329.276.

Les doses utilisables sont variables selon l'affection à traiter. Elles peuvent par exemple varier de 0,1 à 20 mg/kg par jour par voie orale. De préférence, la dose employée varie de 0,1 à 5 mg/kg par jour et est fonction de l'état endocrinien du patient.

Les exemples suivants illustrent l'invention sans toutefois la limiter.

**Exemple 1 : La 4-(hydroxyméthyl) 5-imino-4-méthyl 1-[4-nitro 3-(trifluorométhyl)-phényl] 2-imidazolidinone.**

Stade A : 2-amino 2-(hydroxyméthyl) propane nitrile.

On ajoute 305,5g de sulfate d'ammonium à une solution de 91,2g de cyanure de sodium dans 228 $cm^3$ d'eau déminéralisée et agite la suspension pendant 30 minutes. On refroidit à + 10 C et ajoute en 30 minutes 175g d'hydroxyacétone. On laisse 30 minutes à 10 C puis laisse réchauffer pendant 1 h 30. On ajoute ensuite 300

$cm^3$ d'acétate d'éthyle, agite vigoureusement, amène à sec, reprend les cristaux obtenus dans 700 $cm^3$ d'acétate d'éthyle, agite 30 minutes, essore et rince par 3 fois 150 $cm^3$ d'acétate d'éthyle. On amène les eaux-mères à sec et obtient 170g de produit auquel on ajoute 340 $cm^3$ de dichlorométhane. On agite, amorce la cristallisation, laisse 1 heure à 20 C puis une nuit à 5 C sous agitation et azote. On essore, rince les cristaux obtenus par 40 $cm^3$ puis 2 fois 20 $cm^3$ de dichlorométhane à +10 C.

On obtient 47,6g de cristaux attendus F=74 C.

Stade B : 4-(hydroxyméthyl) 5-imino-4-méthyl 1-[4-nitro 3-(trifluorométhyl)-phényl] 2-imidazolidinone.

On agite 15 minutes une solution de 113,4g d'isocyanate de 3-trifluorométhyl 4-nitro phényl dans 1800 $cm^3$ de dichloroéthane puis ajoute en 5 minutes à 20 C, 45g de 2-amino 2-(hydroxyméthyl) propanenitrile obtenu selon le stade A. On agite une nuit, vide le solvant réactionnel, rince au dichloroéthane et ajoute 2 l de dichloroéthane. On porte au reflux, broie les blocs formés, laisse refroidir à 25 C puis refroidit à 10 C. On essore l'insoluble et rince par 3 fois 100 $cm^3$ de dichloroéthane. On sèche sous vide et obtient 107,5g de cristaux. Le produit cristallisé est broyé puis repris dans 2140 $cm^3$ de dichloroéthane à 10% de méthanol. On porte la suspension au reflux, ajoute du charbon actif à la solution obtenue, filtre à chaud et glace les eaux-mères.

On essore le précipité, le rince par 2 fois 80 $cm^3$ de dichloroéthane à 10% de méthanol, sèche les cristaux obtenus et recueille 32,7g de produit. On distille les eaux-mères sous vide jusqu'à 1 l environ, essore et rince le précipité par 3 fois 25 $cm^3$ de dichloroétane et obtient 51,3g de produit que l'on reprend dans 400 $cm^3$ de dichloroéthane à 10% de méthanol, porte la suspension au reflux, refroidit à 20 C, laisse 15 minutes puis essore le précipité et le rince par 2 fois 40 $cm^3$ de dichloroéthane à 10% de méthanol. On obtient 34,1 g de produit soit en tout 66,8g de produit F = 170 C.

**Exemple 2 : 4-(hydroxyméthyl) 4-méthyl 1-[4-nitro 3-(trifluorométhyl)-phényl] 2,5-imidazolidine dione.**

On porte au reflux une suspension de 69,7g de produit obtenu à l'exemple 1 dans 500 $cm^3$ d'acide chlorhydrique dilué au demi. Une solution partielle apparaît puis un précipité. On maintient 1 h 30 au reflux puis laisse refroidir 2 heures à + 5 C environ.

On essore l'insoluble puis le rince par 7 fois 70 $cm^3$ d'eau déminéralisée (pH final 6-7).

On obtient 67,9g de produit attendu sous forme de cristaux blancs. F = 226 C.

**Exemple 3 : 5-(hydroxyméthyl) 5-méthyl 3-[4-nitro 3-(trifluorométhyl) phényl] 2,4-imidazolidine dione (isomères (+) et (-).**

Stade A : (12R)-(-)-5-[[[9,10,11,12,14,15-hexahydro 9,10 [3′,4′]-furanoanthracèn-12-yl] oxy] méthyl] 5-méthyl 3-(4-nitro 3-trifluorométhylphényl) 2,4-imidazolidine dione et (12R)-(+)-5-[[[9,10,11,12,14,15-hexahydro 9,10 [3′,4′]-furanoanthracèn-12-yl] oxy] méthyl] 5-méthyl 3-(4-nitro 3-trifluorométhylphényl) 2,4-imidazolidine dione.

On porte au reflux pendant 10 minutes sous atmosphère inerte 11 g du produit racémique obtenu à l'exemple 2 dans 500 cm3 de tétrahydrofuranne en présence de 5,5 g d'acide paratoluène sulfonique puis ajoute en 2 heures et demie une solution comprenant 11 g de lactol anthracénique (S) dans 500 cm3 de tétrahydrofuranne. On chauffe 3 heures au reflux, élimine le solvant sous pression réduite, chromatographie le résidu sur silice (éluant : cyclohexane-acétate d'éthyle 1-1 puis flugène-tétrahydrofuranne 7-3). On obtient 1,8 g d'isomère 12R (-), 0,8 g d'isomère 12R (+) et 2,2 g de mélange que l'on chromatographie de nouveau (éluant : flugène-tétrahydrofuranne 7-3) pour obtenir 0,3 g d'isomère 12R (-) et 1 g d'isomère 12R (+) supplémentaire.

Stade B : 5-(hydroxyméthyl) 5-méthyl 3-[4-nitro 3-(trifluorométhyl) phényl] 2,4-imidazolidine dione isomères (+) et (-).

On chauffe au reflux pendant 1 heure et demie 1,378 g d'isomère 12R (+) obtenu au stade précédent dans 21 cm3 de dioxane, 0,3 g d'acide paratoluène sulfonique et 10 cm3 d'eau. On élimine les solvants sous pression réduite, chromatographie le résidu sur silice (éluant : chlorure de méthylène-acétone 8-2) et obtient 0,51 g de produit brut que l'on cristallise dans l'éther. On obtient 0,453 g de l'isomère (+) attendu.

En opérant de facon identique à partir du diastéréoisomère 12R (-) obtenu au stade précédent, on obtient l'isomère (-) attendu.

Isomère (+) : $[alpha]_D$ = +44,5 ± 1,5 (c = 0,75% méthanol)
Analyse : $C_{12}H_{10}F_3O_5N_3$ = 333,226
Calculé : C% 43,25 H% 3,02 N% 12,61 F% 17,10
Trouvé : 43,2 2,9 12,6 17,0
Isomère (-) : $[alpha]_D$ = -40 ± 1,5 (c = 0,8% méthanol)
Analyse :

Calculé : C% 43,25 H% 3,02 N% 12,61 F% 17,10
Trouvé : 43,1 2,9 12,6 17,2

**Etude pharmacologique de la 4-(hydroxyméthyl) 4-méthyl 1-[4 nitro 3-(trifluorométhyl)-phényl] 2,5-imidazolidine dione.**

(Produit A).

Activité anti-androgène chez le rat.

Des groupes de 5 rats mâles pesant 100g environ, castrés 24 heures avant le début du traitement, recoivent par voie sous-cutanée quotidiennement pendant 8 jours le produit à tester A (2, 10 ou 50 mg/kg associé à 0,5 mg/kg de propionate de testostérone (P.T)) sous un volume de 2,5 ml/kg d'huile de sésame contenant 5% d'alcool benzylique). Un groupe d'animaux castrés sert de témoin et reçoit uniquement le solvant. Les animaux sont sacrifiés 24 heures après le dernier traitement ; les vésicules séminales et la prostate sont prélevées et fixées pendant 24 heures dans une solution de formol à 10% dans l'eau, puis disséquées et pesées.

L'augmentation du poids des organes ou l'inhibition de l'effet induit par le propionate de testostérone traduisent respectivement l'activité androgène ou anti-androgène des produits.

Les résultats suivants ont été obtenus :

| GROUPES | Vésicules séminales poids en mg | % d'inhibition du poids des vésicules séminales | prostate ventrale poids en mg | % d'inhibition du poids de la prostate |
|---|---|---|---|---|
| Témoins | 13,6 ± 0,9 | - | 22,4 ± 1,2 | - |
| Propionate de testostérone (P.T) 0,5 mg | 120,7±15,4 | - | 133,9±10,1 | - |
| P.T 0,5 mg + 2 mg de produit A .... | 82,3±10,8 | 35,7 | 121,6± 8,1 | 11 |
| P.T 0,5 mg + 10 mg de produit A .... | 42,8± 6,0 | 72,7 | 70,8±11,8 | 56,6 |
| P.T 0,5 mg + 50 mg de produit A .... | 22,0± 3,2 | 92,1 | 50,9± 5,6 | 74,4 |

Le produit A (décrit à l'exemple 2) présente une bonne activité anti-androgène vis à vis du propionate de testostérone.

**Revendications**

1) Les produits de formule générale I

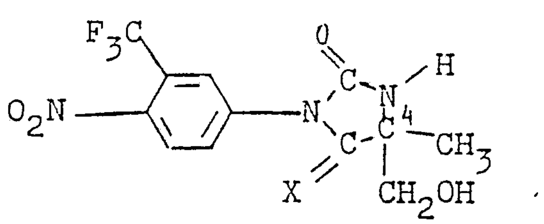

I

dans laquelle X représente un atome d'oxygène ou un groupement imine sous toutes leurs formes racémiques

et optiquement actives possibles.

2) la 4-(hydroxyméthyl) 5-imino 4-méthyl-1-[4-nitro 3-(trifluorométhyl)phényl] 2-imidazolidinone sous forme racémique ou optiquement active.

3) la 4-(hydroxyméthyl) 4-méthyl 1-[4-nitro 3-(trifluorométhyl)-phényl] 2,5-imidazolidine dione sous forme racémique ou optiquement active.

4) la (4R) 4-(hydroxyméthyl) 4-méthyl 1-[4-nitro 3-(trifluorométhyl)-phényl] 2,5-imidazolidine dione.

5) La (4S) 4-(hydroxyméthyl) 4-méthyl 1-[4-nitro 3-(trifluorométhyl)-phényl] 2,5-imidazolidine dione.

6) La 4-(hydroxyméthyl) 4-méthyl 1-[4-nitro 3-(trifluorométhyl)-phényl] 2,5-imidazolidine dione sous forme d'un mélange des isomères 4R et 4S.

7) Procédé de préparation des produits de formule I telle que définie à la revendication 1 caractérisé en ce que l'on fait réagir le 2-amino 2-(hydroxyméthyl) propanenitrile sous forme racémique ou optiquement active avec l'isocyanate de 3-trifluorométhyl 4-nitrophényle pour obtenir un produit de formule I dans laquelle X représente un groupement imino c'est à dire la 4-(hydroxyméthyl 5-imino 4-méthyl 1-[4-nitro 3-(trifluorométhyl) phényl] 2-imidazolidinone sous forme racémique ou optiquement active, produit de formule I dans laquelle X représente un groupement imino sous forme racémique que, si désiré l'on sépare en ses deux isomères optiquement actifs, et produit de formule I dans laquelle X représente un groupement imino sous forme racémique ou optiquement active que, si désiré, on hydrolyse pour obtenir le produit de formule générale I dans laquelle X représente un atome d'oxygène, c'est-à- dire la 4-(hydroxyméthyl) 4-méthyl 1-[4-nitro 3-(trifluorométhyl)phényl] 2,5-imidazolidine-dione sous forme racémique ou optiquement active et produit de formule I dans laquelle X représente un atome d'oxygène sous forme racémique que, si désirée, l'on sépare en ses isomères optiquement actifs.

8) A titre de médicaments les produits de formule I telle que définie à l'une des revendications 1 à 6.

9) Les compositions pharmaceutiques contenant, à titre de principe actif un médicament tel que défini à la revendication 8.

10) A titre de produit industriel nouveau le 2-amino-2-(hydroxyméthyl) propanenitrile sous forme racémique ou optiquement active.

Office européen des brevets

# RAPPORT DE RECHERCHE EUROPEENNE

Numero de la demande

EP 88 40 2102

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-3 651 079 (J.A. SKORCZ & J.T. SUH (COLGATE-PALMOLIVE)<br>* En entier *<br>--- | | C 07 D 233/78<br>C 07 D 233/88<br>A 61 K 31/415 |
| A | EP-A-0 091 596 (CELAMERCK)<br>* En entier *<br>--- | | |
| D,A | FR-A-2 329 276 (ROUSSEL-UCLAF)<br>* En entier *<br>----- | | |
| | | | **DOMAINES TECHNIQUES RECHERCHES (Int. Cl.4)** |
| | | | C 07 D 233/00<br>A 61 K 31/00 |

Le présent rapport a été établi pour toutes les revendications

| Lieu de la recherche | Date d'achèvement de la recherche | Examinateur |
|---|---|---|
| LA HAYE | 15-11-1988 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)